# EUROPÄISCHE PATENTSCHRIFT

(11) **EP 0 248 207 B1**
(45) Veröffentlichungstag und Bekanntmachung des Hinweises auf die Patenterteilung: **03.02.1993**
(21) Anmeldenummer: 87106204.8
(22) Anmeldetag: 29.04.1987
(51) Int. Cl.: C12N 15/76

(54) **Gentamicin-Resistenzgene und ihre Verwendung als Marker**
Genes for gentamicin resistance and its use as a marker
Gènes pour la résistance contre gentamicine et son utilisation comme marqueur

(30) Priorität: 02.05.1986 DE 3614903
(43) Veröffentlichungstag der Anmeldung: 09.12.1987
(73) Patentinhaber: HOECHST AKTIENGESELLSCHAFT, 65926 Frankfurt am Main (DE)
(72) Erfinder: Wohlleben, Wolfgang, Dr., D-4800 Bielefeld (DE); Muth, Günter, D-4800 Bielefeld (DE); Pühler, Alfred, Prof. Dr., D-4800 Bielefeld (DE)

(56) Entgegenhaltungen:
- EP-A- 0 158 872
- CHEMICAL ABSTRACTS, Band 100, Nr. 13, März 1984, Seite 147, Zusammenfassung Nr. 97427p, Columbus, Ohio, US; D.M. SHLAES et al.: "Variability in DNA sequence of closely related nosocomial gentamicin-resistant plasmids", & J. INFECT. DIS. 1983, 148(6), 1013-18
- CHEMICAL ABSTRACTS, Band 104, Nr. 19, Mai 1986, Seiten 371-372, Zusammenfassung Nr. 165227h, Columbus, Ohio, US; V.R. YARULIN et al.: "Use of DNA-DNA hybridization in studies on epidemiology and evolutional similarity of drug resistance determinants", & ANTIBIOT. MED. BIOTEKHNOL. 1986, 31(3), 209-15
- JOURNAL OF ANTIBIOTICS, Band 36, Nr. 10, Oktober 1983, Seiten 1305-1311, Tokyo, JP; T. MURAKAMI et al.: "Cloning of antibiotic-resistance genes in streptomyces"
- PLASMID, Band 12, Juli-Dezember 1984, Seiten 139-141, Academic Press, Inc, New York, US; P.R. HIRSCH et al.: "A physical map of pPH1Jl and pJB4Jl"

## Beschreibung

Für die Klonierung in Streptomyceten stehen bisher nur relativ wenig Resistenzgene zur Verfügung, von denen einige noch besonderen Beschränkungen unterworfen sind. So ist beispielsweise Hygromycin stark toxisch, Viomycin nicht mehr kommerziell erhältlich und Chloramphenicol wegen der bekannten Instabilität des Resistenzgens in Streptomyceten nur in Sonderfällen geeignet. Es besteht somit ein Bedürfnis für weitere Resistenz-Marker.

Viele Streptomyceten sind äußerst sensitiv gegenüber dem Antibiotikum Gentamicin. So wird in der Regel schon bei einer Gentamicin-Konzentration von 0,5 µg/ml das Wachstum auf der Platte gehemmt.

Die Erfindung betrifft nun zwei Gentamicin-Resistenzgene, die in Streptomyceten wirksam und stabil sind und eine Reihe von zur Insertions-Inaktivierung geeigneten Schnittstellen aufweisen.

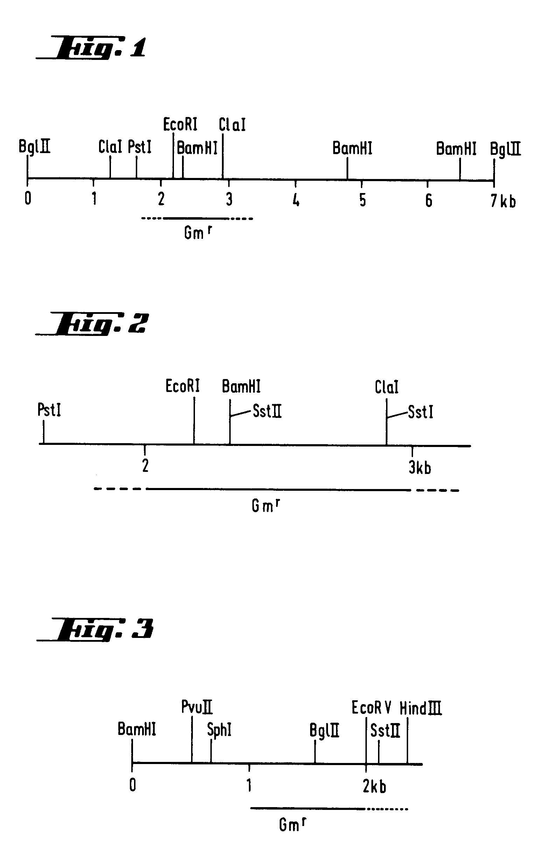

Es wurde gefunden, daß der Streptomyceten-Stamm S. ghanaensis DSM 2932 die ungewöhnlich hohe Dosis von 20 µg/ml Gentamicin toleriert. Dieser Stamm ist in den europäischen Patentanmeldungen mit den Veröffentlichungsnummern 0 158 201 und 0 158 872 genannt. Dieser Stamm enthält das Plasmid pSG5. Es wurde weiterhin gefunden, daß die Gentamicin-Resistenz auf einem etwa 7 kb-BglII-Fragment lokalisiert ist. Durch totale Verdauung der DNA aus dem Stamm DSM 2932, Ligieren der Fragmente in einen geeigneten Vektor und Selektion auf Gentamicin-Resistenz wird der Hybrid-Vektor isoliert, der das 7 kb-Fragment eingebaut enthält. Die Figur 1 zeigt die Restriktionskarte dieses 7 kb-Fragmentes. Die Figur 2 stellt einen Ausschnitt aus der Figur 1 dar, in dem der Kodierbereich näher charakterisiert ist.

Es wurde außerdem gefunden, daß das Plasmid pGM2 (europäische Patentanmeldung 0 158 872, Figur 3) ein besonders gut geeigneter Vektor zur Isolierung des 7 kb-Fragments aus dem Genom von S. ghanaensis DSM 2932 ist. Hierzu wird der Vektor pGM2 mit dem Restriktionsenzym BglII linearisiert und mit dem Enzym alkalische Phosphatase behandelt. Die Gesamt-DNA aus dem Stamm DSM 2932 wird ebenfalls mit BglII vollständig verdaut und ohne Größenfraktionierung mit dem linearisierten Plasmid pGM2 ligiert. Der Ligationsansatz wird in einen geeigneten, Gentamicin-sensitiven Streptomyceten-Stamm transformiert. Nach hinreichend langer Inkubation werden die Regenerationsplatten mit Thiostrepton-haltigem Weichagar überschichtet. Nach weiterer Inkubation werden diese Platten auf Gentamicin-haltigen Agar überstempelt. Hierbei werden Klone isoliert, die sowohl die Thiostrepton-Resistenz aus dem Plasmid pGM2 als auch die Gentamicin-Resistenz aus dem Genom von DSM 2932 enthalten. Eine Plasmidisolierung und Retransformation der Plasmid-DNA in einen Gentamicin-sensitiven Stamm erbringt nur Thiostrepton- und Gentamicin-resistente Kolonien. Durch Restriktionsanalyse wird das 7 kb-Fragment gemäß Figur 1 nachgewiesen.

Als Gentamicin-sensitive Stämme eignen sich S. ghanaensis-Stämme wie ATCC 14672 (US-PS 3 674 866 und 4 621 061), aber auch andere Streptomyceten-Arten wie S. coelicolor, S. lividans, S. prasinus, S. venezuelae und S. geysirensis.

In der Restriktionskarte gemäß Figur 1 sind eine Reihe von Schnittstellen eingezeichnet, darunter singuläre Schnittstellen für PstI und EcoRI. Eine weitere Eingrenzung des Gentamicin-Resistenzgens ist damit möglich. Zur Insertions-Inaktivierung eignen sich BamHI, EcoRI, ClaI, SstI und SstII (Figur 2).

Dieses neue Markergen zeichnet sich nicht nur durch seine vielen nutzbaren Schnittstellen aus, sondern läßt - als streptomyceteneigenes Gen - eine besondere Stabilität der damit versehenen Vektoren erwarten.

Es war bekannt, daß das E. coli-Plasmid pPH1JHI (P.R. Hirsch und J.E. Beringer, Plasmid 12(1984)139-141) ein in E. coli wirksames Gentamicin-Resistenzgen enthält. Überraschenderweise wurde gefunden, daß dieses Resistenzgen in Streptomyceten wirksam und stabil ist. Durch Verdauung mit den Enzymen HindIII und BamHI erhält man ein 2,3 kb-Fragment, auf dem das Resistenzgen liegt (Figur 3).

Auf diesem 2,3 kb-Fragment liegt der zu dem Resistenzgen gehörende Promotor, der nicht nur von RNA-Polymerase gramnegativer Bakterien, sondern unerwarteter Weise auch von Streptomyceten-RNA-Polymerase erkannt wird. Damit ist dieses Gen zur Konstruktion von "Shuttle"-Vektoren (Pendelvektoren) geeignet, die sowohl in gramnegativen Bakterien als auch in Streptomyceten replizieren.

Zur Klonierung mit Insertions-Inaktivierung stehen die Schnittstellen für EcoRV und BglII zur Verfügung.

Das Gentamycin-Resistenzgen aus dem Plasmid pPH1JI wird auch in Corynebakterien exprimiert. Die Formulierung "in Streptomyceten wirksam" soll deshalb keinesfalls so ausgelegt werden, daß diese Wirksamkeit auf Streptomyceten beschränkt ist.

Da die bisher bekannten nativen Streptomyceten-Plasmide keine Resistenz-Marker enthalten, die sich für gentechnologische Anwendungen eignen, eröffnet die Erfindung weitere Möglichkeiten für die Gentechnik mit Streptomyceten-Vektoren.

### Beispiel 1

Das Plasmid pGM2 (europäische Patentanmeldung 0 158 872, S. 3, Absatz 3 und Figur 3) wird mit BglII linearisiert und mit dem Enzym alkalische Phosphatase (aus Kälberdarm) umgesetzt.

Die Gesamt-DNA aus S. ghanaensis DSM 2932 wird mit BglII vollständig verdaut, mit dem pGM2-Verdauungsansatz vereinigt und diesem T4-DNA-Ligase zugesetzt.

Der Ligierungsansatz wird nach S. lividans TK 23 (erhältlich bei der John Innes Foundation, Norwich, England) transformiert (K.F. Chater, D.A. Hopwood, T. Kieser und C.J. Thompson: Gene Cloning in Streptomyces, Current Topics in Microbiol. and Immunol. 96, 69 - 95 (1982)). Nach 18 Stunden werden die Regenerationsplatten mit Thiostrepton-haltigem Weichagar überschichtet. Nach einer Woche werden diese Platten auf Agar überstempelt, der 5 µg/ml Gentamicin enthält. Hierbei wird ein Klon isoliert, der sowohl Thiostrepton- als auch Gentamicin- resistent ist.

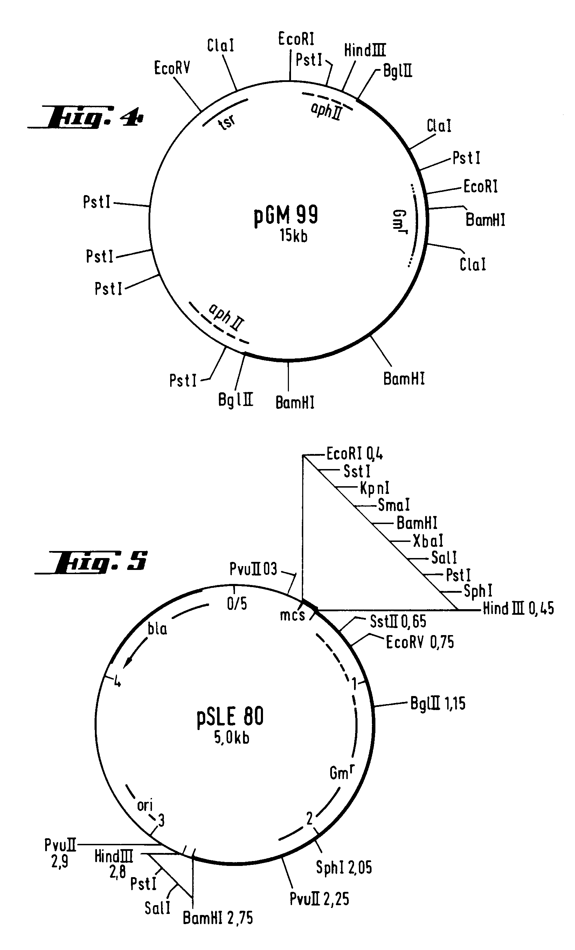

Eine Plasmidisolierung und Retransformation der Plasmid-DNA nach S. lividans TK 23 erbringt nur Thiostrepton- und Gentamicin-resistente Kolonien (193 von 193 gestocherten Kolonien). Die Restriktionsanalyse zeigt eine Insertion des 7 kb-Fragments gemäß Figur 1. Die Figur 4 zeigt das isolierte Plasmid pGM99, das eine Molekülgröße von etwa 15 Kilobasen aufweist und Resistenz gegen Thiostrepton und Gentamicin vermittelt.

### Beispiel 2

Durch Totalverdauung von pPH1JI (Hirsch et al., a.a.O.) mit BamHI erhält man das 3,6 kb-Fragment mit dem Gentamicin-Resistenzgen. Dieses Fragment wird in mit BamHI geöffnetes pUC8 ligiert, wobei das Plasmid R189-2 erhalten wird. Durch Schneiden mit HindIII wird aus diesem Plasmid ein 2,4 kb-Fragment erhalten. Dieses Fragment wird in mit HindIII geöffnetes pUC19 ligiert, wobei das Plasmid pSLE80 (Figur 5) resultiert. Dieses Plasmid ist (in E. coli JM 83) bei der Deutschen Sammlung für Mikroorganismen unter der Nummer DSM 3710 hinterlegt.

Durch Schneiden mit geeigneten Enzymen kann das Gentamicin-resistenzgen reisoliert und kloniert werden.

## Patentansprüche (Patentansprüche für folgende(n) Vertragsstaat(en): BE, CH, DE, FR, GB, GR, IT, LI, LU, NL, SE)

1. Streptomyceten-Vektoren, gekennzeichnet durch in Streptomyceten wirksame Gentamicin-Resistenzgene, die entweder aus S. ghanaensis DSM 2932 durch totale Verdauung mit BgIII, Einbau der Fragmente in ein geeignetes Plasmid und Selektion gegen Gentamicin oder aus dem Plasmid pSLE 80 (DSM 3710) erhältlich sind und deren Gentamicin-Resistenzgene durch die Restriktionskarte gemäß Figur 1 und 2 oder gemäß Figur 3 charakterisiert sind.

2. In Streptomyceten wirksames Gentamicin-Resistenzgen, erhältlich aus S. ghanaensis DSM 2932 durch totale Verdauung mit BglII, Einbau der Fragmente in ein geeignetes Plasmid und Selektion gegen Gentamicin und das durch die Restriktionskarte gemäß Figur 1 und 2 charakterisiert ist.

3. Verwendung der Vektoren nach Anspruch 1 als Streptomyceten-Shuttle-Vektoren.

4. Plasmid pSLE80 (DSM 3710).

## Patentansprüche (Patentansprüche für folgende(n) Vertragsstaat(en): AT, ES)

1. Verfahren zur Herstellung von Vektoren, die in Streptomyceten wirksame Gentamicin-Resistenzgene enthalten, dadurch gekennzeichnet, daß man in diese Vektoren ein Resistenzgen einbaut, das erhältlich ist aus S. ghanaensis DSM 2932 durch totale Verdauung it BgIII, Einbau der Fragmente in ein geeignetes Plasmid und Selektion gegen Gentamicin, oder aus dem Plasmid pSLE80 (DSM 3710), und wobei die bezeichneten Resistenzgene durch die Restriktionskarte gemäß Figur 1 und 2 oder Figur 3 charakterisiert sind.

2. Verfahren nach Anspruch 1, dadurch gekennzeichnet, daß das durch die Restriktionskarte gemäß Figur 1 und 2 charakterisierte Resistenzgen eingebaut wird.

3. Verfahren nach Anspruch 1, dadurch gekennzeichnet, daß das durch die Restriktionskarte gemäß Figur 3 charakterisierte Resistenzgen eingebaut wird.

4. Verfahren nach Anspruch 1,2 oder 3, dadurch gekennzeichnet, daß der Vektor ein Streptomyceten-Shuttle-Vektor ist.

## Claims (Claims for the following Contracting State(s): BE, CH, DE, FR, GB, GR, IT, LI, LU, NL, SE)

1. Streptomyces vectors which have gentamicin-resistance genes active in Streptomyces, which are obtainable either from S. ghanaensis DSM 2932 by total digestion with BglII, incorporation of the fragments into a suitable plasmid and selection using gentamicin or from the plasmid pSLE 80 (DSM 3710), and whose gentamicin-resistance genes have the restriction map shown in Figure 1 and 2 or shown in Figure 3.

2. A gentamicin-resistance gene active in Streptomyces and obtainable from S. ghanaensis DSM 2932 by total digestion with BglII, incorporation of the fragments into a suitable plasmid and selection using gentamicin, and which has the restriction map shown in Figure 1 and 2.

3. The use of the vectors as claimed in claim 1 as Streptomyces shuttle vectors.

4. Plasmid pSLE80 (DSM 3710).

## Claims (Claims for the following Contracting State(s): AT, ES)

1. A process for preparing vectors which contain gentamicin-resistance genes active in Streptomyces, which comprises incorporating into these vectors a resistance gene which is obtainable from S. gahanaensis DSM 2932 by total digestion with BglII, incorporation of the fragments into a suitable plasmid and selection using gentamicin or from the plasmid pSLE80 (DSM 3710), and wherein the said resistance genes have the restriction map shown in Figure 1 and 2 or Figure 3.

2. The process as claimed in claim 1, wherein the resistance gene having the restriction map shown in Figure 1 and 2 is incorporated.

3. The process as claimed in claim 1, wherein the resistance gene having the restriction map shown in Figure 3 is incorporated.

4. The process as claimed in claim 1, 2 or 3, wherein the vector is a Streptomyces shuttle vector.

## Revendications (Revendications pour l'(les) Etat(s) contractant(s) suivant(s): BE, CH, DE, FR, GB, GR, IT, LI, LU, NL, SE)

1. Vecteurs de streptomycètes, caractérisés par des gènes de résistance à la gentamicine actifs dans des streptomycètes et qu'on peut obtenir, soit à partir de S. ghanaensis DSM 2932, par digestion complète avec BglII, insertion des fragments dans un plasmide approprié et sélection dirigée contre la gentamicine, soit à partir du plasmide pSLE 80 (DSM 3710), et dont les gènes de résistance à la gentamicine sont caractérisés par la carte de restriction conforme aux figures 1 et 2 ou à la figure 3.

2. Gène de résistance à la gentamicine actif dans des streptomycètes, qu'on peut obtenir à partir de S. ghanaensis DSM 2932, par digestion complète avec BglII, insertion des fragments dans un plasmide approprié et sélection dirigée contre la gentamicine, et qui est caractérisé par la carte de restriction conforme aux figures 1 et 2.

3. Utilisation des vecteurs selon la revendication 1 comme vecteurs-navettes de streptomycètes.

4. Plasmide pSLE80 (DSM 3710).

## Revendications (Revendications pour l'(les) Etat(s) contractant(s) suivant(s): AT, ES)

1. Procédé pour préparer des vecteurs qui contiennent des gènes de résistance à la gentamicine, actifs dans des streptomycètes, caractérisé en ce qu'on insère un gène de résistance dans ces vecteurs, lequel peut être obtenu, soit à partir de S. ghanaensis DSM 2932, par digestion complète avec BglII, insertion des fragments dans un plasmide approprié et sélection dirigée contre la gentamicine, soit à partir du plasmide pSLE 80 (DSM 3710), et les gènes de résistance indiqués étant caractérisés par la carte de restriction conforme aux figures 1 et 2 ou à la figure 3.

2. Procédé selon la revendication 1, caractérisé en ce qu'on insère le gène de résistance caractérisé par la carte de restriction conforme aux figures 1 et 2.

3. Procédé selon la revendication 1, caractérisé en ce qu'on insère le gène de résistance caractérisé par la carte de restriction conforme à la figure 3.

4. Procédé selon la revendication 1, 2 ou 3, caractérisé en ce que le vecteur est un vecteur-navette de streptomycètes.
